# EUROPÄISCHE PATENTANMELDUNG

(11) **EP 2 311 406 A1**
(43) Veröffentlichungstag der Anmeldung: **20.04.2011**
(21) Anmeldenummer: 10013576.3
(22) Anmeldetag: 11.10.2010
(51) Int. Cl.: A61F 2/08

(54) **Gehörschutzstöpsel**

(30) Priorität: 16.10.2009 DE 202009014015 U
(71) Anmelder: Meyer, Thomas, 91217 Hersbruck (DE)
(72) Erfinder: Meyer, Thomas, 91217 Hersbruck (DE)

(57) **Zusammenfassung**

Es wird ein individuell angepaster Gehörschutzstöpsel für Unterdruckung von Lärm im Ohrkanal vorgeschlagen, der die Nachteile des Standes der Technik vermeidet. Dies wird dadurch erreicht, dass der Gehörschutzstöpsel eine Dämmplastik mit einem weichem Gehörgangszapfen (1) und einer integrierten nahtlos übergehenden Hartkörperschale (2) umfasst.

## Beschreibung

### Gebiet der Erfindung

Die vorliegende Erfindung bezieht sich auf einen Gehörschutzstöpsel der individuell dem Kundenohr mittels einer vorausgehenden Abdrucknahme angepasst wird, welches den Ohrkanal dämmt und vor Lärm schützt.

### Stand der Technik

Der bisher am Markt bestehende Gehörschutz besteht aus hartem oder aus weichem Material, die entsprechenden Dämmmaterialien oder Filter bewirken die entsprechende Dämmung.

Gehörschutzstöpsel aus weichem Material weisen einen angenehmen Tragecomfort auf, werden aber im industriellen Bereich sehr schnell beschädigt oder verunreinigt, oft reißt auch die Kordel aus dem weichen Material.

Harte Gehörschutzstöpsel hingegen erzeugen oft Druckstellen im Gehörgang und können auf Dauer schmerzhaft werden.

### Die Erfindung

Der im Schutzanspruch 1 angegebenen Erfindung liegt das Problem zugrunde, einen Gehörschutzstöpsel zu bauen, welcher einen angenehmen Tragecomfort bei maximaler Abdichtung ermöglicht, aber auch die nötig Stabilität und Sicherheit für den gewerblichenindustriellen Einsatz erfüllt.

**Das Besondere** der Erfindung ist die Kombination von **weichem Gehörgangsstöpsel,** welcher durch Wärme seine Shorehärte verändert. Dieser besteht aus kunststoffähnlichem Material und einer **Hartschale im Conchabereich.**

**Vorteile beim Einsatz als Gehörschutzstöpsel im gewerblichen Bereich:**
- **keine Leckgargenbildung beim kauen oder sprechen**
- **keine Gehörgansweitung im laufe der Zeit, da das Material unter 20 Shore im Gehörgang weich wird**
- **unentfindlich gegen schmutz durch Hartschale und Kunststoffdämmplatte**

Durch spezielles Polymerisationsverfahren wird die Verbindung zwischen Gehörgangsstöpsel und Hartschale hergestellt. Eine Kunststoff Dämmplatte mit Filtereinsatz und Befestigungsvorrichtung für eine Tragekordel schließt den Gehörgangsstöpsel ab, wodurch eine hohe Stabilität und Belastbarkeit erreicht wird.

**Dies ist in dieser Form und Zusammensetzung die erste Gehörschutzotoplastik mit Baumusterprüfbescheinigung des Institutes für Arbeitsschutz und darf somit für den gewerblichen Markt eingesetzt werden.**

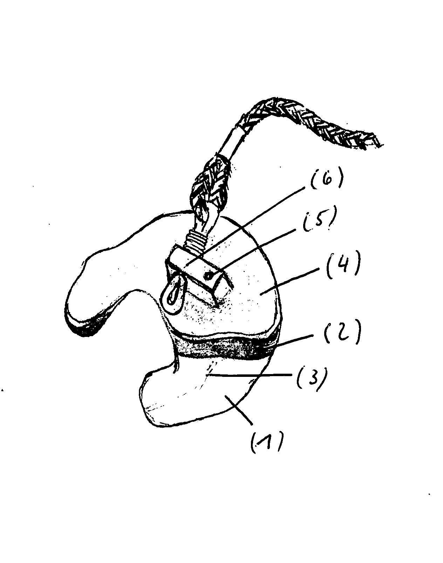

Ein Ausführungsbeispiel der Erfindung wird anhand einer Zeichnung 1-6 erläutert
(1) Weicher Gehörgangszapfen aus kunststoffähnlichem Material
(2) Harter Schalenkörper im Conchabereich aus kunststoffähnlichem Material
(3) Belüftungssystem
(4) Kunststoffdämmplatte
(5) Filtereinsatz
(6) Vorrichtung für die Befestigung der Kordel

## Patentansprüche

1. Individuell Angepasster Gehörschutzstöpsel für Unterdrückung von Lärm im Ohrkanal, welches umfasst: eine Dämmplastik mit kunststoffähnlichen Gehörgangszapfen weich (1), welcher im Ohrkanal untergebracht werden kann um laute Pegel nicht in den Ohrkanal eindringen zu lassen; eine integrierte nahtlos übergehende Hartkörperschale (2) ; eine Belüftungsbohrung (3); eine Kunststoff Dämmplatte (4 ); mit integrierter Filteröffnung ( 5 ) und Befestigungsvorrichtung ( 6 ) der Tragekordel

2. **Gehörgangszapfen, dadurch gekennzeichnet, dass** sich ein spezielles weiches Material durch die Körperwärme dem Gehörgang noch besser Anpasst.

3. **Verbindung mit Hartschalenbereich** , **dadurch gekennzeichnet, dass** die äußerst stabile Verbindung zwischen dem weichen Gehörgangszapfen eine optimale Wärmeableitung darstellt.

4. **Kunststoff Dämmplatte**, **dadurch gekennzeichnet, dass** diese den Gehörschutzstöpsel abschließt und dadurch für äußerste Stabilität und Schmutzunempfindlichkeit sorgt. Der Schall wird durch die Dämmplatte reduziert und gebrochen.

5. **Integrierter Filtereinsatz in der Kunststoffdämmplatte, dadurch gekennzeichnet, dass** entsprechende Filter je nach Anforderung eingesetzt werden können.

6. **Befestigungsvorrichtung für eine Kordel in der Kunststoffdämmplatte, dadurch gekennzeichnet, dass** eine Kordel sicher befestigt werden kann, aber auch so das ab einer bestimmten Zugkraft die Kordel sich selbst löst.

7. Die Kombination aus den Bauteilen 1, 2,4,5 und 6
